# EUROPÄISCHE PATENTSCHRIFT

(11) **EP 0 462 051 B1**
(45) Veröffentlichungstag und Bekanntmachung des Hinweises auf die Patenterteilung: **25.01.1995**
(21) Anmeldenummer: 91810375.5
(22) Anmeldetag: 16.05.1991
(51) Int. Cl.: A61F 2/06

(54) **Verfahren zur Herstellung von mit lebenden Zellen beladenen, porösen, schlauchförmigen Prothesen aus Kunststoff**
Process for manufacturing porous tubular plastic prostheses charged with living cells
Procédé pour fabriquer des prothèses tubulaires poreuses en matière plastique, chargées de cellules vivantes

(30) Priorität: 15.06.1990 CH 2004/90
(43) Veröffentlichungstag der Anmeldung: 18.12.1991
(73) Patentinhaber: SULZER Medizinaltechnik AG, 8404 Winterthur (CH)
(72) Erfinder: Baitella-Eberle, Gabriela, Dr., CH-8700 Küsnacht (CH); Müller-Glauser, Werner, Dr., CH-8542 Wiesendangen (CH); Bisang, Bruno, Dr., CH-8057 Zürich (CH); Huber, August, CH-8352 Räterschen (CH)
(74) Vertreter: Heubeck, Bernhard

(56) Entgegenhaltungen:
- EP-A- 0 248 247
- EP-A- 0 281 736
- EP-A- 0 320 441
- EP-A- 0 363 310
- EP-A- 0 385 925
- WO-A-82/03764

## Beschreibung

Die Erfindung betrifft ein verfahren zur Herstellung von mit lebenden Zellen beladenen, porösen, schlauchförmigen Prothesen aus Kunststoff wie es im Oberbegriff des Anspruch 1 olefiniert wird. Ein solches Verfahren ist aus der EP-A-0 363 310 bekannt.

Für einen erfolgreichen Einsatz als Gefässprothesen müssen poröse Kunststoff-Schläuche - insbesondere wenn es sich um kleinlumige Gefässe mit Durchmessern von etwa 7 mm und kleiner handelt - bekanntlich mit einer möglichst geschlossenen Schicht aus, wenn möglich, patienteneigenen, lebenden Zellen beladen werden, um beispielsweise eine Thrombenbildung und damit einen Verschluss der relativ engen Gefässe oder Gefässprothesen zu verhindern.

Abhängig von der Porenstruktur des Prothesenmaterials haben sich im wesentlichen zwei verfahren für das Beladen der Prothese mit den genannten Zellen und deren Verankerung auf und/oder in der Prothesenwand herausgebildet.

Prothesen mit kleinen Poren von 1 bis 5 »m Durchmesser werden mit derartigen Zellen, die zuvor aus venösen Blutgefässen des Patienten gewonnen und in Kulturen vermehrt worden sind, auf ihrer Oberfläche beschichtet (siehe z.B. EP-A-0 363 310). Die bisher bei diesen Prothesen für das Beladen angewandten Verfahren sind aufwendig und zeitraubend, verursacht durch die notwendige Vermehrungskultur der Endothelzellen.

Prothesen mit grösseren Poren von beispielsweise 20 bis 80 »m Durchmesser werden häufig in textiler Form beispielsweise als Gewebe, Gewirk oder Geflecht hergestellt, wobei die einzelnen Fäden möglicherweise untereinander verklebt sind (CH-Patentanmeldung 00 740/89-9). Bei diesen Prothesen die gegebenenfalls mit Blutplasma und/oder anderem biologischen Material abgedichtet werden, werden die auf bekannte Weise aus Fettgewebe des Patienten gewonnenen Zellen auf der oder in die textile Struktur eingelagert. Es hat sich nun gezeigt, dass die bisher dafür eingesetzten Verfahren eine relativ ungleichmässige Verteilung der Zellen auf bzw. in der Prothese ergeben; darüberhinaus sind bei den bekannten Verfahren die auftretenden Verluste an lebenden Zellen relativ hoch. Schliesslich ist dabei das Risiko von Verunreinigungen relativ gross.

Aufgabe der Erfindung ist es daher, ein Verfahren zum Beladen künstlicher, poröser Gefässprothesen zu entwickeln, das die vorstehend geschilderten Nachteile vermeidet und einfach und zeitsparend ist, so dass das Beladen der Prothese unter Umständen während der Vorbereitung der Implantationsoperation erfolgen kann, und bei dem die Haftung der Zellen auf und/oder in "gross"-porigen Materialien genügend ist.

Diese Aufgabe wird mit der Erfindung durch die Merkmale des Anspruch 1 gelöst.

Die Zentrifugalkräfte bewirken einerseits einen relativ raschen Transport der in einer wässrigen Suspension vorliegenden Zellen auf die und in die poröse Prothesenstruktur hinein, wobei sie besonders das Eindringen der Zellen in die textilen Strukturen fördern.

Mit Vorteil liegt die Prothese während des Zentrifugierens aussen an einer festen Wand an.

Als optimale Zentrifugal-Beschleunigung für eine schonende Ablagerung der Zellen haben sich die in dem Anspruch 1 enthaltenen Werte der 25- bis 50-fachen Erdbeschleunigung erwiesen.

Wie bereits erwähnt, können grossporige Prothesen mit biologischem Material oder Blutplasma oder Fibrien abgedichtet werden. Auch hierfür hat es sich als zweckmässig herausgestellt, wenn das Abdichtmaterial - falls die Prothese vor dem Ablagern der Zellen mit biologischem Material abgedichtet wird - ebenfalls durch Zentrifugieren in die Prothesenwand eingebracht wird, wobei Zentrifugal-Beschleunigungen bis zum 100-fachen der Erdbeschleunigung angewendet werden können. Durch eine vorherige Abdichtung der Prothesenwand wird erreicht, dass die Zellen vorwiegend nahe der inneren Oberfläche der Prothese abgelagert werden. Sollen die Zellen tiefer in der porösen Struktur der Prothese verankert werden, so ist es zweckmässig, die Prothese nach der Zentrifugier-Behandlung für die Zellenverankerung innen und/oder aussen mit biologischem Abdichtmaterial zu tränken, oder die mit der die Zellen enthaltenden Suspension gefüllte Prothese nach dem Zentrifugieren mit biologischem Abdichtmaterial abzudichten.

Neben der Abdichtung kann zusätzlich bei allen der eingangs erwähnten Prothesenarten eine Vorbeschichtung mit mindestens einem Bestandteil der extrazellulären Matrix oder des Blutplasmas erfolgen; die extrazelluläre Matrix besteht bekanntlich aus Fibronektin, Kollagen, Laminin, Elastin oder Thrombospondin bzw. natürlichen oder definierten Gemischen einzelner dieser und weiterer Stoffe. Diese Vorbeschichtung hat wie an sich bekannt, die Aufgabe die Haftung der Zellen auf und in der Prothesenwand zu verbessern.

Um Anwachsen und Abklingen der Wirkung der zentrifugalen Kräfte auf die Zellen in - eine Schädigung der Zellen aus schliessenden - Grenzen zu halten, hat es sich als zweckmässig erwiesen, wenn die Aenderungsgeschwindigkeit der Beschleunigung während des Anlaufens und während des Abklingens mindestens der Rotationen, bei denen lebende Zellen in der Prothese vorhanden sind, einen Wert von 0,5 bis 1 g/sec nicht überschreitet.

Nach dem Zentrifugieren sind in der die Zellen enthaltenden Suspension lediglich noch wenige und/oder tote Zellen vorhanden, die entfernt werden müssen. Ein Absetzen dieser Zellen bis zum Entfernen, beispielsweise durch Entleeren der Prothese und anschliessendes Ausblasen mit Luft, kann verhindert werden, wenn nach Abschluss des Zentrifugierens die mit lebenden Zellen beladene Prothese einer Rotation mit reduzierter Rotationsgeschwindigkeit ausgesetzt wird; dadurch wird gleichzeitig eine gleichmässige Benetzung der abgelagerten Zellen erreicht.

Im folgenden wird die Erfindung anhand von Ausführungsbeispielen näher erläutert.

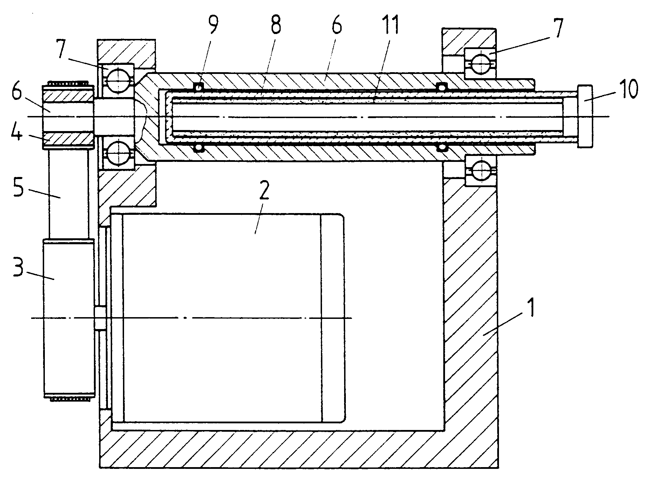

Die einzige Figur zeigt schematisch eine Einrichtung zum Zentrifugieren der Prothese.

Es bestehe die Aufgabe eine Gefässprothese aus einem geflochtenen Kunststoff-Schlauch, deren Herstellung in der EP-A-0 385 925 (veröffentlicht am 05.9.90) beschrieben ist mit Endothel-Zellen zu beladen.

In einem Gehäuse 1 ist ein elektrischer Antriebsmotor 2 gelagert, dessen Drehzahl in bekannter Weise elektronisch geregelt werden kann was nicht ausdrücklich dargestellt ist. Ueber eine Uebersetzung die aus zwei durch einen Zahnriemen 5 verbundenen Zahnrädern 3 und 4 besteht wird die Rotation des Motors 2 auf eine Welle 6 übertragen. Das Uebersetzungsverhältnis im vorliegenden Fall beträgt etwa 1:5. Die Welle 6, die über zwei Kugellager 7 ebenfalls im Gehäuse 1 gelagert ist, ist als Hohlwelle ausgebildet, deren dem Antrieb abgewandtes freies Ende offen ist.

In ihrem Hohlraum kann ein Glas- oder Kunststoffrohr 8 eingeschoben werden, das mit Hilfe von zwei O-Ringen 9 in der Hohlwelle 6 zentriert gehalten wird.

Im Innern des Rohres 8, das mit einem Stopfen 10 aus Kunststoff verschlossen werden kann, ist die zu zentrifugierende Prothese 11 angeordnet. Dies wird einfach in das Rohr 8 eingelegt; sie legt sich beim Steigern der Drehzahl selbständig an die Wand des Rohres 8 an und ist so in diesem fixiert. Der Durchmesser des Glasrohres 8 ist so gewählt, dass die Prothesenaussenwand möglichst eng an der Innenwand des Rohres 8 anliegt.

Für das Füllen und Entleeren des Rohres 8 kann der Stopfen 10 entfernt werden. Es ist jedoch auch möglich, in ihm einen Mutterkonus, wie er bereits früher beschrieben worden ist (CH-PS 671 683), für den Ansatz handelsüblicher Injektionsspritzen vorzusehen.

Nach dem Einlegen der Prothese 11 wird das Rohr 8 mit bidestilliertem Wasser gefüllt und in einem Sterilisations-Autoklaven bei etwa 120^{o}C etwa 20 min lang sterilisiert. Dieser erste Schritt des neuen Verfahrens wird bei allen zu beladenen Prothesen unabhängig von ihren Porengrössen und/oder ihren Materialien durchgeführt.

Bei der Verwendung gewisser Arten, von Prothesen - beispielsweise aus mit Gelatine getränkter, textiler Maschenware oder aus gewissen Materialien, wie z.B. mit Poren versehenem Polyurethan -, ist es unter Umständen zweckmässig, die Prothese in einem zusätzlichen fakultativen Verfahrensschritt vor jeglicher Beschichtung oder Beladung einer Aequilibrierbehandlung zu unterziehen, bei der die äussere und die innere Oberfläche der Prothese oder die Gelatine mit einer Flüssigkeit ins Gleichgewicht gebracht werden, d.h. beispielsweise mit Wasser gesättigt werden, um beispielsweise die Gelatine zu erweichen, die Poren zu öffnen und durchlässig zu machen oder Oberflächenspannungen abzubauen. Für diesen Prozess wird die sterilisierte Prothese bei Raumtemperatur etwa 1 bis 3 Tage in eine unter der Bezeichnung "HBS-2" bekannte, physiologische Pufferlösung eingelegt.

Ein weiterer nicht unbedingt erforderlicher Verfahrensschritt ist die erwähnte Vorbeschichtung mit extrazellulärer Matrix oder Blutplasma. Im vorliegenden Beispiel sei für eine Vorbeschichtung die - bei grossporigen Prothesen - vor oder nach der Abdichtung durchgeführt werden kann, Fibronektin verwendet. Die Beschichtung erfolgt nach einem für die Beschichtung von Kulturgefässen üblichen Verfahren. Im einzelnen wird zunächst die Pufferlösung der Aequilibrierbehandlung aus der Prothese entfernt und diese mit einer Fibronektin-Lösung gefüllt, wobei beispielsweise 28 »g Fibronektin/ml vorhanden sind. Nach einer üblichen Einwirkungszeit der Fibronektin-Lösung auf die Protheseninnenwand von beispielsweise 24 Stunden bei Umgebungstemperatur, ist die Prothese für die Abdichtung und/oder Beladung mit lebenden Zellen bereit. Hierfür haben sich in der Reihenfolge von Beladen und Abdichten unterschiedliche Vorgehen als geeignet herausgestellt.

Während oberflächlich mit Zellen zu beladende oder zu beschichtende Prothesen 11 mit kleinen Poren von 1 bis 5 »m keine Abdichtung ihrer Wände bedürfen, kann eine Abdichtung grossporiger, vor allem textiler Prothesen 11 vor, nach oder während der Beladung mit lebenden Zellen und deren Verankerung erfolgen.

Sollen grossporige Prothesen vor allem an der Oberfläche mit Zellen beladen werden, so empfiehlt es sich, eine Abdichtung vor dem Beladen der Prothese 11 und dem Verankern der Zellen durchzuführen. Für eine vorherige Abdichtung hat sich ein Vorgehen bewährt, bei dem zunächst Blutplasma, das zuvor auf etwa 4^{o}C abgekühlt worden ist, in die ebenfalls auf die genannte Temperatur gekühlte, gegebenenfalls vorbeschichtete Prothese 11 bzw. das Glasrohr 8 eingefüllt wird; dieses wird dann in die Hohlwelle 6 des Motors eingeschoben. Die Drehzahl des Motors wird in einem Zeitintervall von etwa 1 min auf eine Drehzahl von etwa 4 000 U/min gebracht, was bei einer Prothese 11 mit einem Lumen von 5 mm einer Zentrifugal-Beschleunigung an der Prothesenwand von etwa 45-facher Erdbeschleunigung entspricht. Die Drehzahl wird etwa 5 min beibehalten; anschliessend lässt man die Rotation mit einer Drehzahländerung von etwa 67 U/min abklingen, entfernt das Blutplasma aus dem Glasrohr 8 und bläst dieses mit steriler Luft aus.

Nunmehr wird eine Suspension mit lebenden Endothel-Zellen in das Rohr 8 eingefüllt, die pro cm² zu beladender Prothesenoberfläche etwa 10⁵ bis 10⁶ Zellen enthält. Die Prothese 11 rotiert nun mit den gleichen Drehzahlen bzw. Drehzahländerungswerten wiederum 5 min; anschliessend wird sie entleert und wiederum ausgeblasen.

Durch eine geringfügige Variation der Abdichtungsschritte ist es auch bei vorheriger Abdichtung möglich, die Zellen tiefer in die Poren der Prothesenwand zu verankern. Diese Variation besteht darin, dass die Plasma-Füllung aus der Prothese entfernt und diese ausgeblasen wird, ehe die beschriebene, erste Rotation der dann mit Luft gefüllten Prothese 11 durchgeführt wird. Enddrehzahlen und Beschleunigungen bleiben die gleichen wie bei der ersten Variante einer vorherigen Abdichtung.

Eine noch mehr in die Tiefe gehende Beladung und Verankerung von Zellen erhält man, wenn man eine Abdichtung - von innen und/oder von aussen - erst nach der Beladung mit Zellen vornimmt. In diesem Fall wird nach dem bereits geschilderten Rotieren der mit einer Zellsuspension gefüllten Prothese 11 und dem anschliessenden Ausblasen die beladene Prothese 11 - ähnlich wie bei der Variante der vorherigen Abdichtung - zur Abdichtung kurzseitig mit Plasma gefüllt, entleert und ausgeblasen oder aus dem Glasrohr 8 entnommen und manuell in einer Schale mit Blutplasma gewälzt. Auch bei dieser Variante des Verfahrens werden die genannten Drehzahlen und Beschleunigungen angewendet.

Steht für das Beladen der Prothese und das Verankern der Zellen nur relativ wenig Zeit zur Verfügung, so empfiehlt es sich Abdichtung und Zellenverankerung gleichzeitig durchzuführen. Für die wiederum mit gleichen Werten erfolgende Rotation wird in diesem Fall zusammen mit der Zellsuspension heparinisiertes Plasma in die Prothese eingefüllt; heparinisiertes Plasma ist Blutplasma, dem als Anti-Koagulanz Heparin beigemischt ist, wobei die Heparin-Konzentration beispielsweise 500 Units/ml beträgt. Nach dem Zentrifugieren wird die Prothese 11 entleert und mit Protamin-Sulfat gespült, durch das die Wirkung des Heparins aufgehoben wird, so dass eine Koagulation und damit ein Verkleben der Poren der Prothesenwand stattfinden kann. Bei dieser Variante des Verankerungsverfahrens findet wie schon früher beschrieben, nach jedem Entleeren von Flüssigkeit ebenfalls ein Ausblasen der Prothese 11 mit Luft statt.

## Patentansprüche

1. Verfahren zur Herstellung von mit lebenden Zellen beladenen, porösen, schlauchförmigen Prothesen (11) aus Kunststoff, z.B. von kleinlumigen Gefässprothesen, wobei die Prothese (11) eine Rotation um ihre Längsachse erfährt und lebende Zellen in die Prothese (11) eingebracht und in der Prothesenwand bzw. auf ihr abgelagert werden, dadurch **gekennzeichnet**, dass die Zellen durch die Rotation unter Einwirkung einer Zentrifugalkraft auf der Oberfläche und/oder in der Prothesenwand abgelagert werden, wobei die Zentrifugal-Beschleunigung für die Zellen an der Protheseninnenwand während des Zentrifugierens das 25- bis 50-fache der Erdbeschleunigung (g) beträgt.

2. Verfahren nach Anspruch 1, dadurch gekennzeichnet, dass die Prothese (11) während des Zentrifugieren aussen an einer festen Wand anliegt.

3. Verfahren nach einem der Ansprüche 1 oder 2, dadurch gekennzeichnet, dass die Prothese (11) vor dem Ablagern der Zellen mit biologischem Abdichtmaterial abgedichtet wird, das ebenfalls durch Zentrifugieren in die Prothesenwand eingebracht wird, wobei Zentrifugal-Beschleunigungen bis zum 100-fachen der Erdbeschleunigung (g) angewendet werden.

4. Verfahren nach einem der Ansprüche 1 oder 2, dadurch gekennzeichnet, dass die Prothese (11) nach der Zentrifugier-Behandlung für die Zellenverankerung innen und/oder aussen mit biologischem Abdichtmaterial getränkt wird.

5. Verfahren nach einem der Ansprüche 1 oder 2, dadurch gekennzeichnet, dass die Prothese (11) während des Zentrifugierens mit der die Zellen enthaltenden Suspension mit biologischem Abdichtmaterial abgedichtet wird.

6. Verfahren nach einem der Ansprüche 1 bis 5, daduch gekennzeichnet, dass die Prothese (11) mit mindestens einem Bestandteil der extrazellulären Matrix und/oder des Blutplasmas vorbeschichtet wird.

7. Verfahren nach einem der Ansprüche 1 bis 6, dadurch gekennzeichnet, dass die Änderungsgeschwindigkeit der Beschleunigung während des Anlaufens und während des Abklingens mindestens der Rotationen, bei denen lebende Zellen in der Prothese vorhanden sind, einen Wert von 0,5 bis 1 g/sec nicht überschreitet.

8. Verfahren nach einem der Ansprüche 1 bis 7, dadurch gekennzeichnet, dass nach Abschluss des Zentrifugierens die mit lebenden Zellen beladene Prothese (11) einer Rotation mit reduzierter Rotationsgeschwindigkeit ausgesetzt wird.

## Claims

1. A method of production of porous prostheses (11) formed from
flexible plastics tube charged with living cells, e.g., of vascular prostheses of small lumen, in which the prosthesis (11) experiences a rotation about its longitudinal axis and living cells are introduced into the prosthesis (11) and precipitated into or respectively onto the wall of the prosthesis, characterized in that through the rotation the cells are precipitated onto the surface and/or into the wall of the prosthesis under the action of centrifugal force, the centrifugal acceleration of the cells against the inner wall of the prosthesis during centrifugung amounting to 25 to 50 times the acceleration (g) due to gravity.

2. A method as in Claim 1, characterized in that
during centrifuging the outside of the prosthesis (11) rests against a solid wall.

3. A method as in one of the Claims 1 or 2, characterized in that
before the precipitation of the cells the prosthesis (11) is sealed with biological sealing material which is likewise introduced into the wall of the prosthesis by centrifuging, the centrifugal accelerations applied being up to 100 times the acceleration (g) due to gravity.

4. A method as in one of the Claims 1 or 2, characterized in that
after the centrifugal treatment the prosthesis (11) is soaked inside and/or outside with biological sealing material for anchoring the cells.

5. A method as in one of the Claims 1 or 2, characterized in that
during centrifuging with the suspension containing the cells the prosthesis (11) is sealed with biological sealing material.

6. A method as in one of the Claims 1 to 5, characterized in that
the prosthesis (11) is precoated with at least one constituent of the extracellular matrix and/or of the blood plasma.

7. A method as in one of the Claims 1 to 6, characterized in that
during running up and during dying down of at least the rotations during which living cells are present in the prosthesis the rate of change of acceleration does not exceed a value of from 0.5 to 1 g/sec.

8. A method as in one of the Claims 1 to 7, characterized in that
after the conclusion of the centrifuging the prosthesis (11) charged with living cells is subjected to rotation at reduced speed of rotation.

## Revendications

1. Procédé de réalisation de prothèses tubulaires poreuses (11) de matière plastique, chargées de cellules vivantes, par exemple de prothèses de vaisseaux de petite section, suivant lequel la prothèse (11) subit une rotation autour de son axe longitudinal et des cellules vivantes sont introduites dans la prothèse (11) et déposées dans la cloison de la prothèse ou sur elle, caractérisé en ce que les cellules sont déposées sur la surface et/ou dans la cloison de la prothèse par la rotation et donc sous l'effet d'une force centrifuge, l'accélération centrifuge des cellules contre la paroi interne de la prothèse correspondant pendant la centrifugation à 25 à 50 fois l'accélération terrestre (g).

2. Procédé selon la revendication 1, caractérisé en ce que la prothèse (11) est plaquée extérieurement contre une paroi fixe pendant la centrifugation.

3. Procédé selon l'une des revendications 1 et 2, caractérisé en ce qu'avant le dépôt des cellules, la prothèse (11) est imperméabilisée par une matière biologique d'imperméabilisation qui est également placée par centrifugation dans la paroi de la prothèse, l'accélération centrifuge utilisée pouvant atteindre 100 fois l'accélération terrestre (g).

4. Procédé selon l'une des revendications 1 et 2, caractérisé en ce qu'après le traitement de centrifugation, la prothèse (11) est imprégnée intérieurement et/ou extérieurement d'une matière biologique d'imperméabilisation pour la fixation des cellules.

5. Procédé selon l'une des revendications 1 et 2, caractérisé en ce que la prothèse (11) est imperméabilisée par une matière biologique d'imperméabilisation pendant la centrifugation avec la suspension contenant les cellules.

6. Procédé selon l'une des revendications 1 à 5, caractérisé en ce que la prothèse (11) est préalablement enduite d'au moins un composant de la matrice extra-cellulaire et/ou du plasma sanguin.

7. Procédé selon l'une des revendications 1 à 6, caractérisé en ce que la vitesse de variation de l'accélération, pendant la montée en vitesse et pendant la chute de vitesse d'au moins la rotation au cours de laquelle des cellules vivantes sont présentes dans la prothèse, ne dépasse pas une valeur de 0,5 à 1 g/sec.

8. Procédé selon l'une des revendications 1 à 7, caractérisé en ce qu'à la fin de la centrifugation, la prothèse (11) chargée de cellules vivantes est soumise à une rotation à vitesse réduite.
